# EUROPEAN PATENT APPLICATION

(11) **EP 1 619 178 A1**
(43) Date of publication of application: **25.01.2006**
(21) Application number: 05254449.1
(22) Date of filing: 16.07.2005
(51) Int. Cl.: C07C 51/25, C07C 51/215, C07C 57/04, C07C 253/26, C07C 255/08, B01J 23/28, B01J 27/057

(54) **Improved catalytic (Amm) oxidation process for conversion of lower alkanes to carboxylic acids and nitriles**

(30) Priority: 23.07.2004 US 590523 P
(71) Applicant: Rohm and Haas Company, Philadelphia, PA 19106-2399 (US)
(72) Inventor: Cavalcanti, Fernando Antonio Pessoa, Lafayette Hill Pennsylvania 19444 (US)
(74) Representative: Kent, Venetia Katherine

(57) **Abstract**

An improved single-step catalytic (amm)oxidation process for the conversion of one or more C₃ to C₅ alkanes, in the presence of supercritical fluid, to one or more (amm)oxidation products, such as unsaturated carboxylic acids and unsaturated nitriles.

## Description

### Field of the Invention

The present invention relates to an improved single-step catalytic (amm)oxidation process for the conversion of one or more C₃ to C₅ alkanes to one or more (amm)oxidation products, including unsaturated carboxylic acids and unsaturated nitriles, in the presence of supercritical fluid.

### Background of the Invention

Unsaturated carboxylic acids such as acrylic acid and methacrylic acid are industrially important as starting materials for various synthetic resins, coating materials and plasticizers. Nitriles, such as acrylonitrile and methacrylonitrile, are industrially important intermediates for the preparation of fibers, synthetic resins, synthetic rubbers, and the like. Such unsaturated carboxylic acids and nitriles can be produced by catalytic (amm)oxidation of lower (i.e., C₃ to C₅) alkanes and alkenes, such as propane, propene, butane (including n- and iso-butane), butene (including 1-, 2- and iso-butene), pentanes and pentenes.

The currently practiced commercial process for the production of acrylic acid involves conversion of propene in a two-step heterogeneous catalytic vapor phase oxidation reaction, in the presence of suitable mixed metal oxide catalysts. Similarly, the most popular method for producing nitriles is to subject a C₃ to C₅ alkene, such as propene or isobutene, with ammonia and oxygen, to a heterogeneous vapor phase oxidation reaction, in the presence of a suitable mixed metal oxide catalyst. In most cases, the catalyst formulations used in these processes are proprietary to the catalyst suppliers, but the technology is well established.

In view of the lower price of C₃ to C₅ alkanes (for example, propane and isobutane) in comparison to the corresponding C₃ to C₅ alkenes (for example, propene and isobutene), attention has been drawn to the development of catalysts and processes for the production of unsaturated carboxylic acids and unsaturated nitriles by heterogeneous catalytic vapor phase (amm)oxidation reaction, using the cheaper C₃ to C₅ alkanes as the hydrocarbon starting materials. For example, catalysts capable of catalyzing the single-step partial oxidation of propane to acrylic acid in yields up to 48% (see U.S. Patent No. 5,380,933) have been developed and continue to be improved.

In addition, some refinements to the oxidation process itself have been developed and further improvements to the oxidation process continue to be sought and welcomed by industry. For example, it is known to include additional starting materials, including additional reactants, such as molecular oxygen and/or steam, as well as inert materials, such as nitrogen and carbon dioxide to act as diluents or heat moderators, along with the hydrocarbon starting material that is fed to the one-step oxidation process.

Recently, the use of supercritical fluids as reaction media in processes for the oxidation of hydrocarbons have been explored with some success. Supercritical fluids have liquid-like densities and gas-like diffusivities. It has been shown that conducting heterogeneous catalytic vapor phase oxidation reactions in supercritical fluid media, such as supercritical carbon dioxide, water or ethane, to produce oxygenates from hydrocarbons, sometimes results in better oxygenate selectivity and yield with less catalyst damage. Supercritical carbon dioxide is recognized as a particularly environmentally friendly selection for the supercritical fluid. See Kerler, B., et al., *Partial oxidation of propane in compressed carbon dioxide using CoO*_{*x*}/*SiO*_{*2*} *catalysts,* Applied Catalysis A: General 220, Elsevier Science B.V., (2001) 243-252. In particular, Kerler, B., et al. used a CoOₓ/SiO₂ catalyst in supercritical carbon dioxide for partial oxidation of propane in continuous and batch reaction processes. Kerler, B., et al. theorized that supercritical fluid medium provides better heat transfer, thereby reducing development of catalyst "hot spots", and improved mass transport, which prevents over-oxidation of the partial oxidation products by more efficiently removing these products from the catalyst surface. However, partial oxidation products reported by Kerler, B., et al. primarily included propene and methanol, with lesser selectivities (e.g., generally less than 5.3% individually) to acetaldehyde and acrolein and much lesser selectivities (e.g., collectively not more than 4.4 percent) to acrylic acid, acetone, acetic acid and propanol.

Additional reports have issued concerning the partial oxidation of propane using supercritical carbon dioxide, e.g., Kerler, B., et al., *Partial oxidation of alkanes to oxygenates in supercritical carbon dioxide,* Catalysis Today 61, Elsevier Science B.V., (2000) 9-17 and Martin, A., et al., *Partial Oxidation of Propane Using Dense Carbon Dioxide,* Chem. Eng. Technology 24, Wiley-VCH Verlag GmbH, (2001) 41-44, and the partial oxidation of isobutane using supercritical conditions, e.g., Fan, L., et al., *Air oxidation of supercritical phase isobutene to tert-butyl alcohol,* Chem. Commum. (1997) 1179-1180 and Fan, L., et al., *Reaction phase effect on tertiary butyl alcohol synthesis by air oxidation of isobutene,* Applied Catalysis A: General 158, Elsevier Science B.V., (1997) L41-L46.

Notwithstanding the aforesaid findings and studies, there appear to be no reports of successful production of significant quantities of unsaturated carboxylic acids (such as acrylic acid and methacrylic acid) or unsaturated nitriles (such as acrylonitrile and methacrylonitrile) from hydrocarbon starting materials (for example, alkanes, including propane and butane) by conducting heterogeneous catalytic partial oxidation under supercritical conditions. The chemical industry would welcome further improvements to increase the yields of (amm)oxidation processes for the conversion of one or more C₃ to C₅ alkanes to valuable partial (amm)oxidation products, including unsaturated carboxylic acids and nitriles. It is believed that the present invention provides such an improved process by employing supercritical conditions and suitable partial (amm)oxidation catalysts.

### Summary of the Invention

The present invention relates generally to a process for single-step catalytic (amm)oxidation of one or more C₃ to C₅ alkanes to produce one or more (amm)oxidation products, wherein one or more reaction zones are provided, each of which comprises one or more catalysts capable of catalyzing the single-step (amm)oxidation reaction and the process comprises the steps of: (a) feeding at least one supercritical fluid to the one or more reaction zones; (b) maintaining each of the one or more reaction zones at a temperature and a pressure sufficient to keep the at least one supercritical fluid in its supercritical state; (c) feeding the one or more C₃ to C₅ alkanes to at least one of the one or more reaction zones; and (d) contacting the one or more C₃ to C₅ alkanes with the at least one catalyst to form one or more (amm)oxidation products.

In a particular embodiment of the process of the present invention, at least one of the one or more catalysts comprises a mixed metal oxide having the empirical formula:

MoVₐNb_{b}X_{c}Z_{d}Oₙ

wherein X is at least one element selected from the group consisting of Cr, P, Te and Sb; Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements; and wherein a, b, c, d and n represent the molar ratios of V, Nb, X, Z and O, respectively and 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements.

More particularly, the one or more (amm)oxidation products comprise at least one product selected from the group consisting of unsaturated carboxylic acids and unsaturated nitriles and the supercritical fluid comprises at least one material selected from the group consisting of carbon dioxide, water, ammonia, propane, propene, i-butane or i-butene.

In a particular embodiment, the supercritical fluid comprises carbon dioxide, the one or more C₃ to C₅ alkanes comprise at least propane, and the one or more (amm)oxidation products comprise at least (meth)acrylic acid. In another particular embodiment, the process of the present invention further comprising the step of feeding ammonia to the one or more reaction zones and the at least one supercritical fluid comprises carbon dioxide, the one or more C₃ to C₅ alkanes comprise at least propane, and the one or more (amm)oxidation products comprise at least acrylonitrile.

### Detailed Description of the Invention

The present invention provides an improved process for single-step catalytic (amm)oxidation of one or more C₃ to C₅ alkanes, in the presence of at least one supercritical fluid, to produce one or more (amm)oxidation products, including unsaturated carboxylic acids and unsaturated nitriles.

It is noted that the term "(amm)oxidation", as used herein, includes oxidation and ammoxidation reactions and the term "oxidation", as used herein, is intended to include both oxidation and partial oxidation. The term "(amm)oxidation products", as used herein, means and is intended to include the products of oxidation, partial oxidation, and ammoxidation reactions, depending upon the particular starting materials and reaction conditions selected.

Generally, the process of the present invention includes the step of providing one or more reaction zones, each comprising one or more catalysts capable of catalyzing the (amm)oxidation of one or more C₃ to C₅ alkanes to produce one or more (amm)oxidation products. The process further involves feeding suitable starting materials, which comprise one or more C₃ to C₅ alkanes, at least one supercritical fluid, and, optionally, an oxygen-containing gas and/or ammonia, to the one or more reaction zones and maintaining each of the one or more reaction zones at a temperature and a pressure sufficient to keep the at least one supercritical fluid in its supercritical state. As will be explained in further detail hereinafter, the one or more C₃ to C₅ alkanes may also serve as the supercritical fluid. The process in accordance with the present invention comprises the additional step of (amm)oxidizing the one or more C₃ to C₅ alkanes, in the presence of the one or more catalysts and the supercritical fluid, to produce one or more (amm)oxidation products. The one or more (amm)oxidation products comprise at least one product selected from the group consisting of unsaturated carboxylic acids and unsaturated nitriles.

The process of the present invention may be operated as a batch process or a continuous process, as those terms are understood by persons of ordinary skill in the relevant art. The selection of one or more reaction vessels suitable to contain the one or more reaction zones and at least one supercritical fluid is also well within the knowledge and ability of persons having ordinary skill in the relevant art. For example, without limitation, where it is desired to operate the process of the present invention in batch fashion, a stirred batch autoclave would be suitable, and where a continuous process is desired, a continuous flow reactor would be suitable. It is noted that, in either case, suitable reaction vessels must be capable of safely holding and containing the at least one supercritical fluid within a range of temperatures and pressures which are sufficient to maintain the supercritical fluid(s) in their supercritical state. Such reaction vessels are commercially available from various chemical process industry sources. In addition, where the process of the present invention is arranged as a continuous process, it may be practiced in a single pass mode (only fresh feed is fed to the reactor) or in a recycle mode (at least a portion of the reactor effluent is returned to the reactor).

Furthermore, in accordance with the present invention, the process of the present invention may employ one or more reaction vessels, each of which may contain one or more reaction zones. Each of the one or more reaction zones contains at least one suitable catalyst, which may be diluted or undiluted, as is well-known to those of ordinary skill in the art.

Catalysts suitable for use in connection with the process of the present invention are those which are capable of catalyzing the (amm)oxidation reaction of one or more C₃ to C₅ alkanes to produce one or more (amm)oxidation products, such as unsaturated carboxylic acids and unsaturated nitriles. For example, without limitation, mixed metal oxide catalysts comprising molybdenum and vanadium (hereinafter, "Mo/V-based catalysts") are known to be particularly active and selective for such reactions, but are not the only catalysts capable of catalyzing the aforesaid (amm)oxidation reaction.

More particularly, for example, without limitation, catalysts suitable for use in the process of the present invention include those which comprise mixed metal oxides having then following basic empirical formula:

MoVₐNb_{b}X_{c}Z_{d}Oₙ

wherein X is at least one element selected from the group consisting of Cr, P, Te and Sb, Z is at least one element selected from the group consisting of W, Cr,Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, and wherein a, b, c, d and n represent the molar ratios of V, Nb, X, Z and O, respectively, and 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements. Other suitable catalysts include, but are not limited to, mixed metal oxide catalysts which are variants of the above-described catalysts. Additional suitable catalysts include, but are not limited to, the catalysts disclosed in any one of the following patent documents, each of which is hereby incorporated herein by reference: U.S. Patent Nos. 5,498,588, 5,675,057, 5,693,857, 5,696,047, 6,383,978, 6,403,525, 6,407,031, 6,407,280, 6,461,996, 6,472,552, 6,504,053, 6,589,907, 6,624,111, 6,642,174 and 6,646,158.

Generally, the starting materials suitable for use in connection with the single-step (amm)oxidation process of the present invention include, but are not limited to, one or more C₃ to C₅ alkanes, or a mixture of one or more C₃ to C₅ alkanes with one or more corresponding C₃ to C₅ alkenes, at least one supercritical fluid and, optionally, ammonia. Suitable starting materials may also, optionally, include steam, an oxygen-containing gas, and diluent materials.

As used herein, the term "one or more C₃ to C₅ alkanes" means one or more straight chain or branched chain alkanes having from 3 to 5 carbons atoms per alkane molecule, for example, propane, butane and pentane. The term "one or more C₃ to C₅ alkenes" means one or more straight chain or branched chain alkenes having from 3 to 5 carbons atoms per alkene molecule, for example, propene, butane and pentene. As used herein, the term "a mixture of one or more C₃ to C₅ alkanes with one or more corresponding C₃ to C₅ alkenes" means a mixture that includes one or more straight chain or branched chain alkanes having from 3 to 5 carbons atoms per alkane molecule and one or more of the corresponding straight chain or branched chain alkenes having from 3 to 5 carbons atoms per alkene molecule, such as, without limitation, a mixture of propane and propene, or a mixture of n-butane and n-butenes, etc.

The particular one or more C₃ to C₅ alkanes selected as the starting materials for use in connection with the present invention will, of course, depend upon the particular desired (amm)oxidation product or products. For example, when propane or isobutane is used as the starting material alkane, acrylic acid or methacrylic acid will be obtained, respectively, in good yield.

The purity of the one or more C₃ to C₅ alkanes used as the starting material is not particularly limited, and one or more C₃ to C₅ alkanes containing a lower alkane such as methane or ethane, air or carbon dioxide, as impurities, may be used without any particular problem. Further, the starting material alkane(s) may be a mixture of various alkanes. Similarly, where a mixture of one or more C₃ to C₅ alkanes with one or more corresponding C₃ to C₅ alkenes is used as the starting material, the purity of the starting material mixture is not particularly limited, and a mixture of alkane and alkene containing a lower alkene such as ethene, a lower alkane such as methane or ethane, air or carbon dioxide, as impurities, may be used without any particular problem. Further, the starting material mixture may be a mixture of various alkanes and alkenes.

There is no limitation on the source of the one or more C₃ to C₅ alkanes. They may be purchased, per se, or in admixture with an alkene and/or other impurities, as discussed hereinabove. Moreover, the one or more C₃ to C₅ alkanes, regardless of source, and the one or more C₃ to C₅ alkenes, regardless of source, may be blended as desired. For example, without limitation, in a mixture of one or more C₃ to C₅ alkanes with one or more corresponding C₃ to C₅ alkenes, the one or more corresponding C₃ to C₅ alkenes may be present in an amount of at least 0.1 % by volume, including at least 1.0% by volume to 99% by volume, or even 3% by volume to 90% by volume.

Generally, and for purposes of this description of the present invention, the term "supercritical fluid" is used to mean a substance that is in a state at or above their critical temperature (T_{c}) and critical pressure (Pc). As is known by persons practicing in the field of supercritical fluid chemistry, isothermally increasing the pressure of a gas-phase substance which is already above its critical temperature produces a supercritical fluid. Similarly, isobarically increasing the temperature of a liquid-phase substance which is already above its critical pressure produces a supercritical fluid. Such supercritical fluids exhibit properties of both gases and liquids and are, therefore, sometimes beneficially used in various chemical reactions and processes. In the process of the present invention, supercritical fluid is used beneficially as the reaction medium for the single-step catalytic (amm)oxidation of one or more C₃ to C₅ alkanes to produce one or more (amm)oxidation products. For example, without limitation, supercritical carbon dioxide, water, ammonia, propane, propene, i-butane or i-butene, would be suitable for use as the supercritical fluid in connection with the process of the present invention. For purposes of the present invention, one or more of the reactant starting materials, such as the one or more C₃ to C₅ alkanes, may be provided in its supercritical state and fed to the one or more reaction zones and, thereby, serve as both one or more of the reactants, as well as the supercritical fluid, in accordance with the present invention. Supercritical carbon dioxide (T_{c}, = 304K (31 °C) and P_{c}, = 7.37 MPa (1069 psia)) is particularly suitable for use as the supercritical fluid, in view of its substantially inert character and other environmentally friendly characteristics.

There is no particular limitation on the nature and source of the supercritical fluid, although it is most often initially made available at non-supercritical conditions and then heated or pressurized, as appropriate, to reach a supercritical state prior to feeding to the one or more reaction zones of the process of the present invention. Additionally, it is noted that the supercritical fluid may be fed directly to the one or more reaction zones. Alternatively, a supercritical fluid may be mixed or blended with other starting materials, such as the one or more C₃ to C₅ alkanes, or the mixture of one or more C₃ to C₅ alkanes and one or more corresponding C₃ to C₅ alkenes, to form a blended supercritical fluid feed stream which is then fed to the one or more reaction zones. All or a portion of the carbon dioxide fed to the (amm)oxidation process of the present invention may be recycled from downstream units of the (amm)oxidation process, or it may be obtained from other related or unrelated processes, or it may be purchased and supplied independently of other processes.

As will be discussed in further detail hereinafter, when production of unsaturated nitriles from one or more C₃ to C₅ alkanes is desired, the starting materials must also comprise ammonia, in addition to the one or more C₃ to C₅ alkanes and supercritical fluid. The ammonia may be fed to the one or more reaction zones separately from, or at least partially blended with, either or both of the one or more C₃ to C₅ alkanes and supercritical fluid.

The detailed mechanism of the (amm)oxidation reaction of the process of the present invention is not clearly understood, but the (amm)oxidation reaction is carried out by oxygen atoms present in the one or more catalysts in each of the one or more reaction zones, or by molecular oxygen present in the starting materials. Where it is desired to provide molecular oxygen to the (amm)oxidation process, an oxygen-containing gas, such as air, may be included among the starting materials fed to the one or more reaction zones.

As used herein, the term "oxygen-containing gas" means any gas comprising from 0.01% up to 100% oxygen, including, for example, pure oxygen gas, ozone gas, and air, as well as blends of oxygen and/or ozone and one or more other gases such as nitrogen, argon and helium. Other examples of "oxygen-containing gas" include, but are not limited to, nitrogen monoxide (NO), nitrogen dioxide (NO₂) and nitrous oxide (N₂O), as well as their blends with one or more other gases such as oxygen, ozone, nitrogen, argon and helium. Since purity is not particularly required, it is suitable and often most economical to use air as the oxygen-containing gas in the starting materials for the process of the present invention.

For example, when unsaturated carboxylic acids are the desired (amm)oxidation products and it is desired to include molecular oxygen among the starting materials, the proportion of the starting materials fed to each of one or more reaction zones may, for example, be as follows: one or more C₃ to C₅ alkanes, or the mixture of one or more C₃ to C₅ alkanes with one or more corresponding C₃ to C₅ alkenes, in an amount between 0.1 vol% and 50 vol%, such as between 1 vol% and 25 vol%; supercritical fluid in an amount between 0 vol% and 95 vol%, such as between 10 vol% and 90 vol%; oxygen in an amount between 0.1 vol% and 50 vol%, such as between 1 vol% and 25 vol%; and water (steam) in an amount between 0 vol% and 50 vol%, such as 5 vol% and 25 vol%, based upon the total volume of the starting materials fed to each of the one or more reaction zones.

When unsaturated nitriles are the desired (amm)oxidation products and it is desired to include molecular oxygen among the starting materials, the proportion of the starting materials fed to each of one or more reaction zones may be, for example, without limitation, as follows: one or more C₃ to C₅ alkanes, or a mixture of one or more C₃ to C₅ alkanes with one or more corresponding C₃ to C₅ alkenes, in an amount between 0.1 vol% and 50 vol%, such as between 1 vol% and 25 vol%; supercritical fluid in an amount between 0 vol% and 95 vol%, such as between 10 vol% and 90 vol%; ammonia in an amount between 1 vol% and 50 vol%, such as between 1 vol% and 25 vol%; oxygen in an amount between 0.1 vol% and 50 vol%, such as between 1 vol% and 25 vol%; and water (steam) in an amount between 0 vol% and 50 vol%, such as 5 vol% and 25 vol%, based upon the total volume of the starting materials fed to each of the one or more reaction zones.

It is also possible to use only the one or more C₃ to C₅ alkanes, or a mixture of one or more C₃ to C₅ alkanes with one or more corresponding C₃ to C₅ alkenes, along with at least one supercritical fluid, but without an oxygen-containing gas (in other words, substantially in the absence of molecular oxygen), as the starting materials for the (amm)oxidation process in accordance with the present invention. In such a case, it is preferred to adopt a catalyst regeneration method wherein at least a portion of the one or more catalysts of each reaction zone is appropriately withdrawn from each reaction zone from time to time, then sent to an oxidation regenerator, regenerated and then returned to the reaction zone for reuse. For example, the catalyst regeneration may comprise contacting an oxidative gas such as oxygen, ozone, air or a nitrogen oxide with the catalyst in the regenerator usually at a temperature of from 300° to 600°C (573 to 873 K).

Of course, in the (amm)oxidation process of the present invention, it is important that the hydrocarbon (i.e., C₃ to C₅ alkanes) and oxygen concentrations in the feed gases be maintained at the appropriate levels to minimize or avoid entering a flammable regime within each of the one or more reaction zones or, especially, at the outlet of each of the one or more reaction zones. Generally, it is preferred that the outlet oxygen levels be low to both minimize after-burning of (amm)oxidation products and, particularly, in the recycle mode of operation, to minimize the amount of oxygen in the recycled gaseous effluent stream. In addition, maintenance of each of the reaction zone temperatures at a low temperature (e.g., below 450°C (723K)) is extremely attractive because after-burning of the (amm)oxidation products becomes less of a problem, which enables the attainment of higher selectivity to the desired (amm)oxidation products. The catalysts suitable for use with the process of the present invention typically operate more efficiently at the lower temperature range set forth above, significantly reducing the formation of acetic acid and carbon oxides as by-products, and increasing selectivity to the desired (amm)oxidation products, such as acrylic acid.

As a diluent to adjust the space velocity and the oxygen partial pressure, an inert material such as nitrogen, argon, carbon dioxide or helium may be also fed to the one or more reaction zones, for example, as one of the starting materials. It is further noted that where the selected supercritical fluid is inert to the (amm)oxidation reaction (such as carbon dioxide which is substantially inert to the partial oxidation of propane to acrylic acid), the selected supercritical fluid may also function as a diluent in the reaction system.

When production of unsaturated carboxylic acids from one or more C₃ to C₅ alkanes is desired, it is useful to include steam among the starting materials fed to the process. In such a case, as a starting material gas to be supplied to the reaction system, a gas mixture comprising a steam-containing C₃ to C₅ alkane, or a steam-containing mixture of one or more C₃ to C₅ alkanes with one or more corresponding C₃ to C₅ alkenes, and an oxygen-containing gas, is often used. However, the aforesaid steam-containing alkane, or the aforesaid steam-containing mixture of alkane and alkene, and the oxygen-containing gas may be alternately (i.e., separately) supplied to the reaction system. The steam to be employed may be present in the form of steam gas in the reaction system, and the manner of its introduction is not particularly limited.

When steam is supplied together with the one or more C₃ to C₅ alkanes, or the mixture of one or more C₃ to C₅ alkanes with one or more corresponding C₃ to C₅ alkenes, as starting material gas, the selectivity for an unsaturated carboxylic acid is distinctly improved, and the unsaturated carboxylic acid can be obtained from the one or more C₃ to C₅ alkanes, or the mixture of one or more C₃ to C₅ alkanes with one or more corresponding C₃ to C₅ alkenes, as starting material gas, in good yield simply by contacting in one reaction zone (i.e., in one stage). However, the conventional technique utilizes a diluent, such as nitrogen, argon, carbon dioxide or helium, for the purpose of diluting the starting materials. Where the selected supercritical fluid is inert to the (amm)oxidation reaction (such as carbon dioxide is substantially inert to the partial oxidation of propane to acrylic acid), it may also function as a diluent in the reaction system. Such a diluent may be used together with the steam to adjust various process operating parameters, including but not limited to, the space velocity, the oxygen partial pressure and the steam partial pressure.

Further, as a diluent, an inert material such as nitrogen, carbon dioxide, argon or helium may be supplied. The molar ratios of the starting materials, (alkane or mixture of alkane and alkene) : (oxygen) : (diluent) : (H₂O), based upon the total moles of starting materials fed to the one or more reaction zones, is suitably (1) : (0.1 to 10): (0 to 20) : (0.2 to 70), for example, without limitation. (1): (1 to 5.0) : (0 to 10) : (5 to 40).

In accordance with the process of the present invention, the starting materials are fed to the one or more reaction zones as follows. The at least one supercritical fluid is fed to the one or more reaction zones such that each reaction zone contains supercritical fluid in contact with the one or more catalysts contained therein. Each of the one or more reaction zones is maintained at a temperature and a pressure sufficient to keep the at least one supercritical fluid in its supercritical state using conventional, well-known means, such as, but not limited to, heat exchangers, metering vessels and high-pressure pumps and compressors. The particular temperatures and pressures required to keep the at least one supercritical fluid in its supercritical state will depend upon the particular supercritical fluids used, for example, if carbon dioxide is used as the supercritical fluid, the required temperature would be at least 304K (31 °C) and the required pressure would be at least 7.37 MPa (1069 psia). The critical temperature and pressure points for a given fluid are determinable empirically or by calculation, or (for pure, unmixed fluids) consulting available informational sources such as the CRC Handbook of Chemistry and Physics, published by CRC Press, and such determination methods are well-known to persons of ordinary skill in the art. See also, for example, Kerler, B., et al., *Partial oxidation of propane in compressed carbon dioxide using CoO*_{*x*}/*SiO*_{*2*} *catalysts,* Applied Catalysis A: General 220, Elsevier Science B.V., (2001) 243-252, at 246-247.

Either simultaneously, or subsequent to, the feeding of the supercritical fluid, the one or more C₃ to C₅ alkanes are fed to the one or more reaction zones, whereupon the one or more C₃ to C₅ alkanes come into contact with the one or more catalysts and are converted to one or more (amm)oxidation products. As already discussed above, the one or more C₃ to C₅ alkanes may be fed separately and independently from the supercritical fluid, as separate feed streams under sub- or supercritical conditions, or they may be at least partially blended with one another prior to feeding them to at least one of the one or more reaction zones. Additionally, the one or more C₃ to C₅ alkanes may be fed at supercritical conditions and, thereby, also serve as the supercritical fluid for the process.

Furthermore, the one or more C₃ to C₅ alkanes may be fed to more than one reaction zone and such reaction zones need not be contiguous or adjacent with one another. Similarly, where nitriles are to be produced and ammonia is included among the starting materials, the ammonia may be fed separately and independently from the supercritical fluid and one or more C₃ to C₅ alkanes, as a separate feed stream under sub- or supercritical conditions, or the ammonia may be at least partially blended with one or more of the other starting materials, prior to feeding the starting materials to at least one of the one or more reaction zones. Moreover, the ammonia may be fed to more than one reaction zone, together with or independently of the other starting materials, and such reaction zones need not be contiguous or adjacent with one another.

Where the supercritical fluid is not selected from among the reactant starting materials and the one or more C₃ to C₅ alkanes are fed separately from the supercritical fluid, the one or more C₃ to C₅ alkanes may be fed to one or more reaction zones. For example, in an embodiment where a total of three reaction zones arranged in series with one another are provided, the one or more C₃ to C₅ alkanes could be fed to the first and last (i.e., third) reaction zones. Alternatively, the one or more C₃ to C₅ alkanes could be fed only to the second reaction zone, or to all three reaction zones. The foregoing discussion also applies to feeding the ammonia to the one or more reaction zones.

In addition, the supercritical fluid may be fed to one of the one or more reactions zones or to multiple reaction zones, as long as, at least initially, the supercritical fluid is provided to each of the one or more reaction zones simultaneously or prior to feeding the one or more C₃ to C₅ alkanes so that the supercritical fluid is available as the reaction medium for the (amm)oxidation reaction. Of course, there are many variations concerning the number and arrangement of reaction zones, as well as to which zones the various starting materials are fed, either together or separately from one another. All such variations are within the ability of persons having ordinary skill in the art to develop and each such variation is within the contemplation and scope of the present invention.

It is noted that the compositions and proportion of the starting materials fed to each of the one or more reaction zones need not be the same as one another in order to realize the benefits of the present invention and, in fact, may need to be different from one another, as will be readily understood and determinable by persons of ordinary skill in the art.

General conditions for the process of the present invention are as follows: the reaction temperature can vary from 200°C to 700°C (473K to 973K), for example, without limitation, 300°C to 400°C (573K to 673K); the gas space velocity, SV, in the vapor phase reaction is usually within a range of from 100 to 10,000 hr⁻¹, preferably 300 to 6,000 hr⁻¹, more preferably 300 to 2,000 hr⁻¹; the average contact time with the catalyst can be from 0.01 to 10 seconds or more, but is usually in the range of from 0.1 to 10 seconds, preferably from 0.2 to 6_seconds. The suitable pressure rarige for each of the one or more reaction zones will be determined by the nature of the selected supercritical fluid and is determinable by persons of ordinary skill in the art. In a single pass mode process, it is preferred that the oxygen be supplied from an oxygen-containing gas such as air. The single pass mode process may also be practiced with oxygen gas addition. In the practice of the recycle mode process, oxygen gas by itself is the preferred source so as to avoid the build up of inert gases in the one or more reaction zones.

A first embodiment of the present invention will now be described, in further detail, wherein propane (as the one or more C₃ to C₅ alkanes), supercritical carbon dioxide (as the supercritical fluid), and air (as the oxygen-containing gas) are provided as the starting materials and the propane is partially oxidized to produce acrylic acid. The process may be a batch process or a continuous process and the catalyst may be contained in the reaction system apparatus in a fixed-bed or a fluidized-bed and may be diluted or undiluted.

In the production of acrylic acid from propane by the process of the present invention, the proportion of the starting materials fed to each of one or more reaction zones is suitably as described hereinabove in connection with the general process as applied to conversion of one or more C₃ to C₅ alkanes to one or more carboxylic acids.

The proportion of air to be supplied to the process is important for the selectivity of the partial oxidation reaction to form acrylic acid, and it is usually at most 25 moles, preferably from 0.2 to 18 moles per mole of propane, whereby high selectivity for acrylic acid can be obtained. With respect to other C₃ to C₅ alkanes, such as isobutane, or to mixtures of C₃ to C₅ alkanes with corresponding C₃ to C₅ alkenes, such as propane and propene, the composition of the feed gas may be selected in accordance with the conditions for propane.

Typical reaction conditions known to persons of ordinary skill in the art, and as discussed hereinabove, for the partial oxidation of propane, or isobutane, to acrylic acid, or methacrylic acid, respectively, may be utilized in accordance with the present invention. For example, the reaction temperature can vary from 200°C to 700°C (473K to 973K), but is usually in the range of from 200°C to 550°C (473K to 823K), more preferably 250°C to 480°C (523K to 753K), most preferably 300°C to 400°C (573K to 673K); the gas space velocity, SV, in the vapor phase reaction is usually within a range of from 100 to 10,000 hr⁻¹, preferably 300 to 6,000 hr⁻¹, more preferably 300 to 2,000 hr⁻¹; the average contact time with the catalyst can be from 0.01 to 10 seconds or more, but is usually in the range of from 0.1 to 10 seconds, preferably from 0.2 to 6 seconds; the suitable pressure range for each of the one or more reaction zones will be determined by the nature of the selected supercritical fluid and is determinable by persons of ordinary skill in the art.

When the partial oxidation reaction of propane, and especially the partial oxidation reaction of propane and propene, is conducted by the process of the present invention, carbon monoxide, carbon dioxide, acetic acid, etc. may be produced as co-products, in addition to acrylic acid. Further, in the process of the present invention, an unsaturated aldehyde may sometimes be formed depending upon the reaction conditions. For example, when propane is present in the starting material mixture, acrolein may be formed; and when isobutane is present in the starting material mixture, methacrolein may be formed. In such a case, such an unsaturated aldehyde can be converted to the desired unsaturated carboxylic acid by subjecting it again to catalytic oxidation in the presence of a suitable oxidation catalyst, as discussed hereinabove, or a conventional oxidation reaction catalyst for an unsaturated aldehyde.

A second embodiment of the process of the present invention will now be described, in further detail, wherein propane (as the one or more C₃ to C₅ alkanes), supercritical carbon dioxide (as the supercritical fluid), ammonia, and air (as the oxygen-containing gas) are provided as the starting materials and the propane is ammoxidized to acrylonitrile. The reaction system apparatus may comprise a fluid- or a fixed-bed catalytic reactor system.

In the production of unsaturated nitriles, such as, but not limited to, acrylonitrile, it is preferred to employ a C₃ to C₅ alkane, such as propane, butane, isobutane, or pentane, as the one or more C₃ to C₅ alkanes in the starting materials. However, in view of the industrial application of nitriles to be produced, it is particularly beneficial to employ a lower alkane having 3 or 4 carbon atoms, particularly propane or isobutane.

Similarly, where it is desired to employ a mixture of one or more C₃ to C₅ alkanes with one or more corresponding C₃ to C₅ alkenes in the starting materials, it is preferred to employ a mixture of a C₃ to C₅ alkane with a corresponding C₃ to C₅ alkene, such as, without limitation, a mixture of propane and propene, butane and butene, isobutane and isobutene, and pentane and pentene. However, in view of the industrial application of nitriles to be produced, it is particularly beneficial to employ a mixture of a lower alkane having 3 or 4 carbon atoms and a corresponding lower alkene having 3 or 4 carbon atoms, such as, without limitation, propane and propene or isobutane and isobutene. Preferably, in the mixture of alkane and alkene, the alkene is present in an amount of at least 0.1% by volume, such as at least 1.0% by volume to 99% by volume, or even from 3% by volume to 90% by volume.

The proportion of air to be supplied for the ammoxidation reaction is important with respect to the selectivity for the resulting acrylonitrile. For example, high selectivity for acrylonitrile is obtained when air is supplied within a range of at most 25 moles, particularly 1 to 15 moles, per mole of the propane. The proportion of ammonia to be supplied for the reaction is preferably within a range of from 0.2 to 5 moles, particularly from 0.5 to 3 moles, per mole of propane. The reaction pressure will be determined by the nature of the particular supercritical fluid that is utilized and is readily determinable by persons having ordinary skill in the art. With respect to other C₃ to C₅ alkanes, such as isobutane, or to mixtures of C₃ to C₅ alkanes with corresponding C₃ to C₅ alkenes, such as propane and propene, the composition of the feed gas may be selected in accordance with the conditions for propane.

This process for production of acrylonitrile from propane, in accordance with the present invention as discussed hereinabove, may be conducted at a temperature of, for example, from 250°C to 600°C (523K to 873K), such as, for example, from 350°C to 550°C (623K to 823K). The gas space velocity, SV, in the gas phase reaction is usually within the range of from 100 to 10,000 hr⁻¹, preferably from 300 to 6,000 hr⁻¹, more preferably from 300 to 2,000 hr⁻¹. As a diluent, for adjusting the space velocity and the oxygen partial pressure, an inert gas such as nitrogen, argon, carbon dioxide, or helium can be employed. It is further noted that where the selected supercritical fluid is inert to the (amm)oxidation reaction (such as carbon dioxide is substantially inert to the partial oxidation of propane to acrylic acid), it may also function as a diluent in the reaction system. When ammoxidation of propane is conducted by the method of the present invention, in addition to acrylonitrile, carbon monoxide, carbon dioxide, acetonitrile, hydrocyanic acid, acrylic acid and acrolein may form as co-products.

The discussion and descriptions provided herein are intended to acquaint others skilled in the art with the present invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not with reference to the above description, but should instead be determined with reference to the appended claims, along with the full scope of equivalents to which such claims are entitled.

### Examples

A suitable catalyst to be used in the following examples is prepared in a manner similar to the synthesis procedure disclosed in U.S. Patent No. 6,642,174. More particularly, a catalyst of nominal composition

Mo_{1.0}V_{0.3}Te_{0.23}Nb_{0.17}Pd_{0.01}Oₓ

is prepared as follows: 200 mL of an aqueous solution containing ammonium heptamolybdate tetrahydrate (1.0M Mo), ammonium metavanadate (0.3M V) and telluric acid (0.23M Te) is formed by dissolving these salts in water at 70°C, and is added to a 2000mL rotavap flask. Then 200 mL of an aqueous solution of ammonium niobium oxalate (0.17M Nb), palladium nitrate hydrate (0.01 M Pd), oxalic acid (0.155M) and nitric acid (0.24M) are added thereto. After removing the water via a rotary evaporator with a warm water bath at 50°C and 28mm Hg, the solid materials are further dried in a vacuum oven at 25°C overnight and then calcined.

Calcination is effected by placing the solid materials in an air atmosphere and then heating them to 275°C at 10°C/min and holding them under the air atmosphere at 275°C for one hour; the atmosphere is then changed to argon and the material is heated from 275°C to 600°C at 2°C/min and the material is held under the argon atmosphere at 600°C for two hours.

The catalyst, thus obtained, is ground with a Freezer/Mill (Model 6750 from Spex CertiPrep™), pressed and sieved to 14 - 20 mesh granules for reactor evaluation.

### Example 1

An exemplary batch process for conversion of propane to acrylic acid by single-step catalytic partial oxidation in supercritical carbon dioxide (CO₂), in accordance one particular embodiment of the present invention as described hereinabove, is performed as follows.

A stirred batch autoclave reactor is provided and the aforesaid catalyst (of nominal composition Mo_{1.0}v_{0.3}Te_{0.23}Nb_{0.17}Pd_{0.01}Oₓ) is filled in a wire basket and fixed in the autoclave reactor. The catalyst is used diluted or undiluted.

Starting materials propane and supercritical CO₂ are each fed, separately, to the autoclave reactor. Propane is fed from a gas cylinder and supercritical CO₂ is dosed by means of a cryostat and a HPLC pump up to the respective partial pressure needed.

The autoclave reactor is heated to the desired reaction temperature. Then, the oxidation reaction is initiated by depressurizing the desired quantity of air (either naturally-occurring, or a synthetic mixture of about 80% nitrogen and 20% oxygen) from a metering tube of a defined volume into the reactor.

The batch reaction is carried out at a pressures of from 2 MPa to 15 MPa, reaction times of from 1 to 5 hours, temperatures of from 473 to 723 K and a molar ratio of supercritical (CO₂) : (air) : (propane) = (90-130) : (2-10) : (1).

It is expected that the foregoing process will produce one or more oxidation products, including acrylic acid.

### Example 2

An exemplary continuous process for conversion of propane to acrylic acid by single-step catalytic oxidation in supercritical carbon dioxide (CO₂), in accordance another particular embodiment of the present invention as described hereinabove, is performed as follows.

A continuous flow reactor is provided and contains the aforesaid catalyst (of nominal composition Mo_{1.0}V_{0.3}Te_{0.23}Nb_{0.17}Pd_{0.01}Oₓ) in a fixed-bed or a fluid-bed arrangement therein. The catalyst is used diluted or undiluted.

Starting materials supercritical CO₂ and propane are dosed (i.e., fed to the continuous flow reactor) in a combined liquid feed stream, by a HPLC pump via a cryostat from a gas cylinder containing 1 % propane in CO₂. Air (either naturally-occurring, or a synthetic mixture of about 80% nitrogen and 20% oxygen) is fed, separately from the combined feed stream, to the continuous flow reactor by a mass flow controller. Both streams are preheated and mixed in a reactor oven module before further flowing, via a pneumatically driven multiport valve, either through a bypass or the reactor. After constant reaction concentrations are achieved, the reaction is initiated by switching the multiport valve from bypass to reactor mode, so that the starting materials are fed to the reactor and contact the catalyst. The product stream is depressurized by a valve which is also used as a back pressure regulator.

The continuous flow reaction is carried out at a pressures of from 2 MPa to 15 MPa, residence times of from 0.1 to 5 seconds , temperatures of from 473 to 723 K and a molar ratio of (CO₂) : (air) : (propane) = (90-130) : (2-10) : (1).

It is expected that the foregoing process will produce one or more oxidation products, including acrylic acid.

### Example 3

An exemplary batch process for conversion of propane to acrylonitrile by single-step catalytic partial oxidation in supercritical carbon dioxide (CO₂), in accordance one particular embodiment of the present invention as described hereinabove, is performed as follows.

A stirred batch autoclave reactor is provided and the aforesaid catalyst (of nominal composition Mo_{1.0}V_{0.3}Te_{0.23}Nb_{0.17}Pd_{0.01}Oₓ) is filled in a wire basket and fixed in the autoclave reactor. The catalyst is used diluted or undiluted.

Starting materials propane, ammonia and supercritical CO₂ are each fed, separately, to the autoclave reactor. Propane is fed from a gas cylinder and ammonia and supercritical CO₂ are dosed by means of a cryostat and a HPLC pump up to the respective partial pressure needed.

The autoclave reactor is heated to the desired reaction temperature. Then, the oxidation reaction is initiated by depressurizing the desired quantity of air (either naturally-occurring, or a synthetic mixture of about 80% nitrogen and 20% oxygen) from a metering tube of a defined volume into the reactor.

The batch reaction is carried out at a pressures of from 2 MPa to 15 MPa, reaction times of from 1 to 5 hours, temperatures of from 473 to 823 K and a molar ratio of supercritical (CO₂) : (air) : (propane) : (ammonia) = (90-130) : (2-10) : (1) : (1-5).

It is expected that the foregoing process will produce one or more (amm)oxidation products, including acrylonitrile.

### Example 4

An exemplary continuous process for conversion of propane to acrylonitrile by single-step catalytic oxidation in supercritical carbon dioxide (CO₂), in accordance another particular embodiment of the present invention as described hereinabove, is performed as follows.

A continuous flow reactor is provided and contains the aforesaid catalyst (of nominal composition Mo_{1.0}V_{0.3}Te_{0.23}Nb_{0.17}Pd_{0.01}Oₓ) in a fixed-bed or a fluid-bed arrangement therein. The catalyst is used diluted or undiluted.

Starting materials supercritical CO₂ and propane are dosed (i.e., fed to the continuous flow reactor) in a combined liquid feed stream, by a HPLC pump via a cryostat from a gas cylinder containing 1% propane in CO₂. Air (either naturally-occurring, or a synthetic mixture of about 80% nitrogen and 20% oxygen) and ammonia are fed, separately from the combined feed stream, to the continuous flow reactor by individual mass flow controllers. The streams are preheated and mixed in a reactor oven module before further flowing, via a pneumatically driven multiport valve, either through a bypass or the reactor. After constant reaction concentrations are achieved, the reaction is initiated by switching the multiport valve from bypass to reactor mode, so that the starting materials are fed to the reactor and contact the catalyst. The product stream is depressurized by a valve which is also used as a back pressure regulator.

The continuous flow reaction is carried out at a pressures of from 2 MPa to 15 MPa, residence times of from 0.1 to 5 seconds , temperatures of from 473 to 823 K and a molar ratio of (CO₂) : (air) : (propane) : ammonia = (90-130) : (2-10) : (1) : (1-5).

It is expected that the foregoing process will produce one or more (amm)oxidation products, including acrylonitrile.

## Claims

1. A process for single-step catalytic (amm)oxidation of one or more C₃ to C₅ alkanes to produce one or more (amm)oxidation products, wherein one or more reaction zones are provided, each of which comprises one or more catalysts capable of catalyzing the single-step (amm)oxidation reaction, said process comprising the steps of:
(a) feeding at least one supercritical fluid to the one or more reaction zones;
(b) maintaining each of the one or more reaction zones at a temperature and a pressure sufficient to keep the at least one supercritical fluid in its supercritical state;
(c) feeding the one or more C₃ to C₅ alkanes to at least one of the one or more reaction zones; and
(d) contacting the one or more C₃ to C₅ alkanes with the at least one catalyst to form one or more (amm)oxidation products.

2. The process of Claim 1, wherein at least one of the one or more catalysts comprises a mixed metal oxide having the empirical formula:
MoVₐNb_{b}X_{c}Z_{d}Oₙ
wherein X is at least one element selected from the group consisting of Cr, P, Te and Sb; Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements;
and wherein a, b, c, d and n represent the molar ratios of V, Nb, X, Z and O, respectively and 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements.

3. The process of Claim 2, wherein the one or more (amm)oxidation products comprise at least one product selected from the group consisting of unsaturated carboxylic acids and unsaturated nitriles.

4. The process of Claim 2, wherein the at least one supercritical fluid comprises at least one material selected from the group consisting of carbon dioxide, water, ammonia, propane, propene, i-butane or i-butene.

5. The process of Claim 2, wherein the at least one supercritical fluid comprises the one or more C₃ to C₅ alkanes.

6. The process of Claim 2, wherein the at least one supercritical fluid comprises carbon dioxide, the one or more C₃ to C₅ alkanes comprise at least propane, and the one or more (amm)oxidation products comprise at least (meth)acrylic acid.

7. A process for the production of one or more unsaturated carboxylic acids by single-step catalytic partial oxidation of one or more C₃ to C₅ alkanes, wherein one or more reaction zones are provided, each of which comprises one or more catalysts capable of catalyzing the single-step partial oxidation reaction, each of said one or more catalysts comprising comprises a mixed metal oxide having the empirical formula:
MoVₐNb_{b}X_{c}Z_{d}On
wherein X is at least one element selected from the group consisting of Cr, P, Te and Sb; Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements;
and wherein a, b, c, d and n represent the molar ratios of V, Nb, X, Z and O, respectively and 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements;
said process comprising the steps of:
(a) feeding at least one supercritical fluid to the one or more reaction zones;
(b) maintaining each of the one or more reaction zones at a temperature and a pressure sufficient to keep the at least one supercritical fluid in its supercritical state;
(c) feeding the one or more C₃ to C₅ alkanes to at least one of the one or more reaction zones; and
(d) contacting the one or more C₃ to C₅ alkanes with the at least one catalyst to form one or more carboxylic acids.

8. The process of Claim 7, wherein the one or more C₃ to C₅ alkanes comprise at least propane and the one or more carboxylic acids comprise at least (meth)acrylic acid.

9. A process for the production of one or more unsaturated nitriles by single-step catalytic ammoxidation of one or more C₃ to C₅ alkanes, wherein one or more reaction zones are provided, each of which comprises one or more catalysts capable of catalyzing the single-step ammoxidation reaction, each of said one or more catalysts comprising comprises a mixed metal oxide having the empirical formula:
MoVₐNb_{b}X_{c}Z_{d}Oₙ
wherein X is at least one element selected from the group consisting of Cr, P, Te and Sb; Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements;
and wherein a, b, c, d and n represent the molar ratios of V, Nb, X, Z and O, respectively and 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements;
said process comprising the steps of:
(a) feeding at least one supercritical fluid to the one or more reaction zones;
(b) maintaining each of the one or more reaction zones at a temperature and a pressure sufficient to keep the at least one supercritical fluid in its supercritical state;
(c) feeding the one or more C₃ to C₅ alkanes to at least one of the one or more reaction zones;
(d) feeding ammonia to at least one of the one or more reaction zones; and
(e) contacting the one or more C₃ to C₅ alkanes and ammonia with the at least one catalyst to form one or more unsaturated nitriles.

10. The process of Claim 9, wherein the one or more C₃ to C₅ alkanes comprise at least propane and the one or more unsaturated nitriles comprise at least (meth)acrylonitrile.
